# EUROPEAN PATENT APPLICATION

(11) **EP 4 759 936 A1**
(43) Date of publication of application: **17.06.2026**
(21) Application number: 24219963.6
(22) Date of filing: 13.12.2024
(51) Int. Cl.: C12P 21/02, C12Q 1/00

(54) **HUMAN CELL-FREE SYSTEM FOR HIGH-THROUGHPUT SCREENING FOR NEW COMPOUNDS FOR TARGETED PROTEIN DEGRADATION**

(71) Applicant: Fraunhofer-Gesellschaft zur Förderung der angewandten Forschung e.V., 80686 München (DE)
(72) Inventor: Buntru, Matthias, 52074 Aachen (DE); Hahnengress, Nils, 52074 Aachen (DE)
(74) Representative: Kuttenkeuler, David

(57) **Abstract**

The present invention relates to a cell lysate wherein the lysis buffer comprises at least one of magnesium acetate, phosphorylation inhibitor K3L, an agonist of dephosphorylation GADD34 and/or a caspase inhibitor for use in, or as, a cell-free system and a method for preparing a cell-free protein synthesis system. Further provided is a method for expressing a protein in the cell-free protein synthesis system, a method for screening for a candidate compound for an effect on a target molecule and a use of the cell-free protein synthesis system. The present invention also provides a kit for the above-mentioned cell-free systems.

## Description

### FIELD OF THE INVENTION

The present invention relates to a cell lysate for use in, or as, a cell-free system and a method for preparing a cell-free protein synthesis system. Further provided is a method for expressing a protein in the cell-free protein synthesis system, a method for screening for a candidate compound for an effect on a target molecule and a use of the cell-free protein synthesis system. The present invention also provides a kit for the above-mentioned cell-free systems.

### DESCRIPTION

Many diseases are not caused by infections through viruses or microorganisms, but are caused by a combination of genetic, physiological, environmental and behavioural factors. Examples for such diseases are cancer, diabetes or certain cardiovascular conditions. Thousands of autologous proteins play a major role in the development and the clinical manifestation of these diseases and are therefore potential therapeutic targets for drug discovery. Despite the discovery of these therapeutic targets, most of these proteins have remained untranslated. A major challenge in developing therapeutics against these targets is that 85% of the human proteome are considered "undruggable", or intractable to traditional drug discovery efforts. These proteins lack easily identifiable binding pockets or catalytic active sites that can be functionally and selectively targeted with a small-molecule, antibody, or other therapeutic modality (1).

These undruggable proteins are for example protein complexes that are involved in scaffolding non-enzymatic functions and may not possess deep binding pockets that can be targeted to disrupt or stabilize protein interactions. Furthermore, also the identification of binding pockets that can be pharmacologically targeted with small molecules within intrinsically disordered proteins remains challenging (2).

Targeted protein degradation (TPD) is currently the most promising approach for degrading these undruggable proteins. TPD uses small compounds like PROteolysis TArgeting Chimeras (PROTACs) and molecular glues to recruit disease-causing proteins for rapid destruction mainly via the endogenous ubiquitin-proteasome system (UPS). PROTACs regulate protein function by degrading target proteins instead of inhibiting them, providing more sensitivity to drug-resistant targets and a greater chance to affect the non-enzymatic functions. Therefore, PROTACs show better selectivity compared to classic inhibitors (3).

PROTACs are hetero bifunctional molecules that connect a target protein ligand to an E3 ubiquitin ligase recruiting ligand with an optimal linker. The degradation is initiated when PROTACs promote the target protein and E3 to form a ternary complex. Followed by a subsequent target protein ubiquitination by bringing the ubiquitination machinery in close proximity and recognition and degradation of the ubiquitinated target protein by the 26S proteasome, which is part of the ubiquitin-proteasome system (UPS) in eukaryotic cells (4).

PROTACs ally with the UPS system to achieve a regulation of protein levels in cells and represent therefore a chemical knockdown strategy with rapidity and reversibility. This strategy results in a new and different biology compared to other gene editing tools by avoiding misinterpretations that arise from potential genetic compensation and/or spontaneous mutations (5). PROTAC technology may also include molecular glues (6), LYTAC (7), PhotoPROTAC (8) AUTAC (9), and Homo-PROTAC (10). PRTOACs have been widely explored throughout the world and have outperformed not only in cancer diseases, but also in immune disorders, viral infections, inflammatory and neurodegenerative diseases.

New PROTACs were designed from a known target-binding ligand. The designing often requires the crystal structure of the protein of interest (POI) ligand bound to its target or its structure-activity relationship (SAR) information. Therefore, it is possible to improve these molecules by finding spots for the linker attachment in areas that are not involved in the target binding. However, the lack of a well-characterized ligand capable of interacting with the desired target limits early-stage drug discovery projects, involving novel biological targets with poorly understood pharmacology (11, 12).

Currently, most PROTACs use small molecules as target warheads. However, small molecule PROTACs also have certain disadvantages in TPD, such as insufficiency of available POI ligand and difficulty in synthesis (13, 14). Small molecule PROTACs heavily rely on the binding pockets of targets. In contrast, peptide ligands are less difficult to develop relative to small molecules while avoiding the restriction of shallow binding pockets through large interacting surfaces between POI and peptides. Many targets that cannot be targeted by small molecules are degraded by peptide PROTACs (15).

New peptide-based ligands can be developed by computational methods (16), phage display (17) and yeast display (18). The new ligand can be fused to a known ligand for an E3 ligase and a POI, respectively, by a linker. However, there is no guarantee that a binder that connects components of the machinery will cause degradation of the POI (19).

Molecular glues are difficult to identify and even harder to design in a rational manner. Most molecular glues have been found serendipitously or are developed retrospectively using a chemical scaffold based upon a known glue (20). Existing methods currently have low throughput, are expensive, and can take 3-5 months to conduct thoroughly (21). Historically, initial biochemical assays have been used to assess activity and rank compounds in small-molecule drug discovery screening programs. However, this has been difficult for molecular glues, as their actions lead to the stabilization of protein-protein interactions or TPD, which rely on the successful formation of ternary complexes that can initiate a sequence of cellular reactions (22).

Currently, there is still a lack of screening technologies for monitoring protein-protein interactions or TPD in a rational and high-throughput manner in the context of the cellular environment (23). New ligands binding to a POI as well as ternary complex formation was investigated by surface plasmon resonance (SPR) (24).

Scholes *et al.* (2021) reviewed different proteomics-based methods to investigate TPD in cells including expression proteomics ("global proteomics"), dual-fluorescence reporters, dual-luminescence reporters and multiplexed dynamic proteomics profiling. They also described a cell-based method to assess POI ubiquitination called MS-based ubiquitinome profiling (25).

Chini (2014) demonstrated a library screening method for molecular glues involving a yeast two-hybrid approach (26).

US2018/0237847 A1 discloses the use of cell-free in vitro transcription/translation systems as rapid prototyping platforms for the synthesis, modification and identification of natural products (NPs), natural product analogues (NPAs) and secondary metabolites. An active UPS or the screening of TPD compounds is not included.

US2022/236273 A1 discloses cell-based screening methods for the identification of peptide ligands for E3 ligases using human HEK293 cells. But the use of cell-free in vitro transcription/translation is not disclosed.

CN112194729 A discloses a probe for the detection of UPS degradation activity, which is also used for the screening of ubiquitin kinase activators and proteasome inhibitors. The use of a cell-free in vitro transcription/translation system as a screening system is not disclosed.

WO23197084 A1 discloses cell-based methods using human cells to identify new effectors for the degradation (e.g. E3 ligases) and stabilization (e.g. deubiquitinases) of proteins at the proteome level. Here too, the use of a cell-free in vitro transcription/translation system as a screening system is not disclosed.

Despite all above progress, so far there are no fast and cost-effective methods to find new degrader compounds like PROTACs and molecular glues in a high-throughput manner. Membrane-impermeable compounds are lost during cell-based screening processes that could represent promising target protein degradation compounds. Additionally, these candidate membrane-impermeable compounds can be further modified for pharmaceutical use to generate membrane permeability.

Thus, it is an object of the invention to provide a high-throughput screening tool for new TPD compounds, which is fast and cost-effective.

### BRIEF DESCRIPTION OF THE INVENTION

Generally, and by way of brief description, the main aspects of the present invention can be described as follows:

In **a first aspect,** the invention pertains to a method for the preparation of a cell lysate for use in, or as, a cell-free system, the method comprises the steps of:
**(a)** cultivating and harvesting human cells;
**(b)** optionally, washing said cultured human cells with a washing buffer;
**(c)** admixing the cells with a lysis buffer, wherein the lysis buffer comprises one or more components selected from the group consisting of:
   (i) magnesium acetate, preferably in a concentration range between 1 mM and 3 mM;
   (ii) an inhibitor of phosphorylation of eIF2α;
   (iii) an agonist of dephosphorylation of eIF2α; and
   (iv) a caspase inhibitor;
**(d)** lysing the cells by incubating the cells in the admixture of step (c), and preferably applying physical stress to the cells, to obtain a crude cell lysate;
**(e)** purifying the crude cell lysate, such as by removing cellular debris from the crude cell lysate, for example by centrifugation and/or filtration and thereby obtaining an aqueous cell lysate;
**(f)** optionally, cryoprotecting the aqueous cell lysate obtained in (e), for example by admixing the aqueous cell lysate with trehalose, preferably in a concentration range between 1.25 and 5 % (w/v).

In **a second aspect,** the invention pertains to a method of preparing a cell-free protein synthesis system, the method comprises a step of providing the aqueous cellular lysate according to the invention, and adding to the aqueous cellular lysate at least:
**(a)** nucleotide triphosphates (NTPs);
**(b)** an amino acid mixture;
**(c)** one or more of the following components (i) to (v):
   (i) magnesium acetate, preferably in a concentration range between 1 mM and 3 mM;
   (ii) an inhibitor of phosphorylation of eIF2α;
   (iii) an agonist of dephosphorylation of eIF2α;
   (iv) a caspase inhibitor;
   (v) a deubiquitinases (DUB) inhibitor, preferably DUB inhibitor PR619; and/or
**(d)** a nucleic acid construct encoding a protein to be expressed with the cell-free protein synthesis system.

In **a third aspect,** the invention pertains to a cell-free system obtained by, or obtainable by, performing a method according to the invention.

In **a fourth aspect,** the invention pertains to a method for expressing a protein, the method comprising the steps of:
**(a)** adding a nucleic acid construct suitable for expressing the protein with the cell-free protein synthesis system according to the invention;
**(b)** isolating the protein encoded by the nucleic acid construct.

In **a fifth aspect,** the invention pertains to a method for screening a candidate compound for an effect on a target molecule, the method comprising:
(a) contacting a candidate compound and a target molecule in a cell-free system according to the invention;
(b) determining the amount or level of effected target molecule over time compared to a control or reference,

wherein the effect on the target molecule is selected from the group consisting of a reversible post translational modification, ubiquitin-proteasome dependent protein degradation, lysosome-dependent protein degradation, inhibition of the ubiquitin-proteasome dependent protein degradation, and RNA degradation,
preferably ubiquitin-proteasome dependent protein degradation, wherein an increased or decreased protein stability or protein degradation compared to the control or reference indicates that the candidate compound is a compound having an effect on ubiquitin-proteasome dependent protein degradation of the target molecule.

In **a sixth aspect,** the invention pertains to a use of the cell-free protein synthesis system according to the invention for protein expression and/or for investigating ubiquitin-proteasome dependent protein degradation, inhibition of the ubiquitin-proteasome dependent protein degradation, protein stabilization, protein phosphorylation, protein dephosphorylation, protein acetylation, lysosome-dependent protein degradation, or RNA degradation.

In **a seventh aspect,** the invention pertains to a cell-free system kit, wherein the kit comprises:
**(a)** an aqueous cellular lysate according to the invention;
**(b)** optionally, additional compounds such as nucleotide triphosphates (NTPs), amino acids, magnesium acetate, GADD34, K3L, caspase inhibitor, DUB inhibitor, creatine phosphate, potassium acetate, DTT, creatine kinase, spermidine, m⁷GpppG cap analogue, T7 polymerase;
**(c)** and, optionally, instructions directing a user to combine the aqueous cellular lysate and the compounds from the kit with a nucleic acid construct encoding a target protein or a peptide PROTAC.
wherein the aqueous cellular lysate and the compounds are disposed in one or more separate containers.

In **an eighth aspect,** the invention pertains to a cell-free system, comprising the following components:
(a) magnesium acetate, preferably in a concentration range between 1 mM and 3 mM;
(b) an inhibitor of phosphorylation of eIF2α;
(c) an agonist of dephosphorylation of eIF2α; and/or
(d) a caspase inhibitor;
wherein the cell-free system is prepared from a cell lysate and comprises a functional transcription/translation capability for protein expression and/or a functional ubiquitin-proteasome system for protein degradation.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

In **the first aspect,** the invention pertains to a method for the preparation of a cell lysate for use in, or as, a cell-free system, the method comprises the steps of:
(a) cultivating and harvesting human cells;
(b) optionally, washing said cultured human cells with a washing buffer;
(c) admixing the cells with a lysis buffer, wherein the lysis buffer comprises one or more components selected from the group consisting of:
   (i) magnesium acetate, preferably in a concentration range between 1 mM and 3 mM;
   (ii) an inhibitor of phosphorylation of eIF2α;
   (iii) an agonist of dephosphorylation of eIF2α; and
   (iv) a caspase inhibitor;
(d) lysing the cells by incubating the cells in the admixture of step (c), and preferably applying physical stress to the cells, to obtain a crude cell lysate;
(e) purifying the crude cell lysate, such as by removing cellular debris from the crude cell lysate, for example by centrifugation and/or filtration and thereby obtaining an aqueous cell lysate;
(f) optionally, cryoprotecting the aqueous cell lysate obtained in (e), for example by admixing the aqueous cell lysate with trehalose, preferably in a concentration range between 1.25 and 5 % (w/v).

In view of the results as obtained in the experiments as performed in the context of the present invention, the inventors identified an improved method for the preparation of a cell lysate. The preparation method included the admixing of the lysis buffer with magnesium acetate, preferably in a concentration range between 1 mM and 3 mM, an inhibitor of phosphorylation of eIF2α, an agonist of dephosphorylation of eIF2α, and a caspase inhibitor.

These additions led to an advantageous gentle method in order to keep the triggering of stress reactions (phosphorylation of elF2alpha, activation of caspase signaling cascades) as low as possible. Caspases can, among other things, inactivate the proteasome and cleave translation factors.

Preferred is the method according to the present invention, wherein the human cells are human embryonic kidney (HEK) cells, preferably HEK293T cells.

Further preferred is the method according to the present invention, wherein the inhibitor of phosphorylation of eIF2α is a pseudosubstrate for an eIF2α kinase, preferably K3L; optionally wherein the K3L is used in a concentration range between 0.01 mg/mL and 0.03 mg/mL.

Preferred is the method according to the present invention, wherein the agonist of dephosphorylation of eIF2α is growth arrest and DNA damage-inducible protein (GADD34), preferably in a concentration range between 0.01 mg/mL and 0.02 mg/mL.

The addition of magnesium acetate and the accessory proteins GADD34 and K3L to the lysis buffer has several advantages. Magnesium plays a major role in many steps of protein synthesis, for example (i) the aminoacylation of tRNA, (ii) formation of the ternary complex between aminoacylated initiator methionine tRNA, GTP, and initiation factor 2, and (iii) ribosomal complex maintenance. GADD34 and K3L can inhibit the phosphorylation of eIF2α.

K3L acts as a pseudosubstrate for eIF2α kinases and prevents phosphorylation of eIF2α. GADD34 recruits a phosphatase PP1c to elF2 to dephosphorylate eIF2α. Therefore, GADD34 and K3L were added to the lysis buffer to keep the phosphorylation of eIF2α caused by cell stress during the disruption procedure as low as possible.

In embodiments herein, the magnesium concentration of the cell lysate may be adjusted by an additional magnesium compound. In some embodiments the additional magnesium compound is a salt; for example, magnesium chloride, magnesium acetate, and magnesium glutamate. For coupling transcription and translation, a sufficient amount of a magnesium salt may be added to the lysate to raise the final magnesium concentration to a level where RNA is transcribed from DNA, and RNA is translated into protein.

Preferred is the method according to the present invention, wherein the lysis buffer comprises at least component (iv), preferably (i) and (iv), more preferably (i), (ii) and (iv) and most preferably components (i) to (iv).

Preferred is the method according to the present invention, wherein the caspase inhibitor comprises a broad spectrum caspase inhibitor, preferably a caspase-1 inhibitor, a caspase-3 inhibitor, a caspase-7 inhibitor, a caspase-9 inhibitor, and/or a caspase-12 inhibitor; more preferably a caspase-3 inhibitor, a caspase-6 inhibitor and/or a caspase-7 inhibitor, for example wherein the caspase-1 inhibitor is selected from the group consisting of YVAD-CHO, VX-740 and VX-765; and/or wherein the caspase-3 inhibitor is selected from the group consisting of Ac-DEVD-CHO and Z-DEVD-FMK; and/or wherein the caspase-7 inhibitor is Q-VD-OPh; and/or wherein the caspase-9 inhibitor is Z-LEHD-FMK; and/or wherein the caspase-12 inhibitor is Z-ATAD-FMK.

Preferred is the method according to the present invention, wherein the Ac-DEVD-CHO (caspase-3/DEVDase inhibitor) is used in a concentration range between 10 µM and 30 µM, preferably between 15 µM and 25 µM.

The addition of caspase inhibitors prevents caspase signaling cascades as they can induce apoptosis, which can lead to phosphorylation of eIF2α. In addition, caspase activation can cause the cleavage of three specific subunits of the 19S regulatory complex of the proteasome and thus the inhibition of proteasomal degradation.

The term "purified" as used herein refers to material that has been isolated under conditions that reduce or eliminate the presence of unrelated materials, i.e., contaminants, including native materials from which the material is obtained.

In the context of the present invention the term "cellular debris" shall mean cell membranes, cell nuclei, non-lysed cells.

Cell membranes may be disrupted in some embodiments by techniques including, for example mechanical disruption, liquid homogenization, enzymatic digestion, high frequency sound waves, decompression, freeze/thaw cycles, and manual grinding.

Preferred is the method according to the present invention, wherein the physical force is selected from the group consisting of pressure, freezing and thawing, sonication, impact forces and shearing forces, such as sieving.

Preferred is the method according to the present invention, wherein the applying physical force comprises sieving the cells to mesh/sieve and/or passing the cells through a tip of a needle cannula.

The applying of physical force by sieving the cells to mesh/sieve and/or passing the cells through a tip of a needle cannula is an advantageous gentle method to reduce stress reactions in the cells and to maintain valuable systems such as the ubiquitin-proteasome system.

Preferred is the method according to the present invention, wherein the mesh/sieve comprises an average pore size of 100 nm to 50 µm, preferably 500 nm to 25 µm, most preferably 1 to 10 µm (such as about 6 µm).

Preferred is the method according to the present invention, wherein the needle cannula has a diameter of less than 2 mm, preferably of less than 1 mm, more preferably of less than 0.5 mm.

Preferred is the method according to the present invention, wherein the method comprises passing the cells through the mesh/sieve and/or needle cannula at least once, preferably at least twice, more preferably for 5 times or more (such as about 7 times through a mesh/sieve and/or about 10 times for a needle cannula).

Preferred is the method according to the present invention, wherein the lysis buffer further comprises ATP, preferably in a concentration range between 1.5 mM and 2.5 mM.

Preferred is the method according to the present invention, wherein the lysis buffer further comprises glycerol, preferably in a concentration range between 1.5 and 2.5 % w/v.

In **a second aspect,** the invention pertains to a method of preparing a cell-free protein synthesis system, the method comprises a step of providing the aqueous cellular lysate according to the invention, and adding to the aqueous cellular lysate at least:
(a) nucleotide triphosphates (NTPs);
(b) an amino acid mixture;
(c) one or more of the following components (i) to (v):
   (i) magnesium acetate, preferably in a concentration range between 1 mM and 3 mM;
   (ii) an inhibitor of phosphorylation of eIF2α;
   (iii) an agonist of dephosphorylation of eIF2α;
   (iv) a caspase inhibitor;
   (v) a deubiquitinases (DUB) inhibitor, preferably DUB inhibitor PR619; and/or
(d) a nucleic acid construct encoding a protein to be expressed with the cell-free protein synthesis system.

Preferred is the method according to the present invention, wherein the nucleic acid construct (d) is added to a final concentration in the cell-free protein synthesis system of 12 nM to 28 nM, preferably 14 nM to 26 nM, more preferably 16 nM to 24 nM, even more preferably 18 nM to 22 nM.

Preferred is the method according to the present invention, further comprising a step of adding creatine phosphate, preferably in a concentration range of 15 mM to 45 mM, more preferably 20 mM to 40 mM, even more preferably 25 mM to 35 mM.

Preferred is the method according to the present invention, wherein the method according to the invention, further comprising a step of adding one or more of:
(a) potassium acetate;
(b) a reducing agent, preferably dithiothreitol (DTT) and/or tris(2-carboxyethyl)phosphine (TCEP);
(c) creatine kinase;
(d) spermidine;
(e) m⁷GpppG cap analogue; and/or
(f) T7 polymerase.

In **a third aspect,** the invention pertains to a cell-free system obtained by, or obtainable by, performing a method according to the invention.

Preferred is the method according to the present invention, wherein the system is a cell-free protein synthesis system and comprises:
**(a)** an aqueous cellular lysate prepared with a method according to the invention;
**(b)** nucleotide triphosphates (NTPs);
**(c)** an amino acid mixture;
**(d)** an active ubiquitin-proteasome system;
**(e)** one or more of the following components (i) to (v):
   (i) magnesium acetate, preferably in a concentration range between 1 mM and 3 mM;
   (ii) an inhibitor of phosphorylation of eIF2α;
   (iii) an agonist of dephosphorylation of eIF2α;
   (iv) a caspase inhibitor;
   (v) a deubiquitinases (DUB) inhibitor, preferably DUB inhibitor PR619, and/or
**(f)** a nucleic acid construct encoding a protein to be expressed with the cell-free protein synthesis system.

Additional components may also be added to the cell-free protein synthesis system in particular embodiments, as desired for improving the efficiency or stability of the synthesis reaction.

In addition to the above components, other materials (such as those specifically utilized in protein synthesis) may be added to the cell-free protein synthesis system as described herein. Such materials may include, for example, other salts, folinic acid, cyclic AMP, inhibitors of protein or nucleic acid degrading enzymes, RNasin, inhibitors or regulators of protein synthesis, adjusters of oxidation/reduction potential(s), reducing agents, chloramphenicol, non-denaturing surfactants, buffer components (such as may be used in the solution to stabilize the reaction pH), PEG, Triton X-100, spermine, spermidine, and putrescine.

In the context of the present invention, the term "cell-free protein synthesis system" refers to an *in vitro* transcription-translation (IVTT) system. These terms have the same meaning and are used interchangeably.

Embodiments herein may be adapted to utilize any human cellular lysate. Human cell-free lysates retain a variety of post-translational processing activities. Human cellular lysates also support the translation *in vitro* of a wide variety of viral and other prokaryotic RNAs, as well as eukaryotic mRNAs. Template mRNAs that have a codon usage that deviates from that of the organism from which the cell lysate is derived may be used efficiently, for example, by supplementing the cell-free protein synthesis system with rare tRNAs and/or amino acids in the organism.

In **a fourth aspect,** the invention pertains to a method for expressing a protein, the method comprising the steps of:
(a) adding a nucleic acid construct suitable for expressing the protein with the cell-free protein synthesis system according to the invention;
(b) isolating the protein encoded by the nucleic acid construct.

The cell-free protein synthesis system as described herein may be used in a method for *in vitro* synthesis of peptides, for example peptide PROTAC. *In vitro* synthesis refers to the cell-free synthesis of biological macromolecules in a reaction mix comprising biological extracts and/or defined reagents. Using the cell-free protein synthesis system herein, a cell-free synthesis reaction may be performed in batch, continuous flow, and semi-continuous flow configurations, as these configurations are known in the art. In some embodiments, batch-cultured cells may be used. In some embodiments, cells may be grown continuously in a stirred-tank fermenter to ensure a reproducible supply of homogeneous cell material.

There are differences between using a static IVTT reaction, versus a continuous or flow-through reactions, that may be a consideration in some applications. For example, the continuous cell-free protein synthesis system is generally used for large-scale industrial production of proteins, whereas static cell-free protein synthesis system reactions are better suited to small scale *in vitro* translations, for example in a research setting.

Additionally, in view of the results as obtained in the experiments as performed in the context of the present invention, the inventors identified that the improved human cell-free *in vitro* transcription-translation (IVTT) system with active UPS is a tool for high-throughput screening for new TPD compounds.

Cell-free protein synthesis systems offer significant advantages over living cell cultures. The advantages are for example 1) no sterile culture is necessary, 2) no cell line development required, 3) ready-to-use system, 4) shorter design-build-test cycles, 5) open system allows precise adjustment of reaction conditions, 6) no cloning required, 7) no loss of non-membrane-permeable compounds in the screening process and 8) lower costs.

The amount of protein expressed with the cell-free protein synthesis system can be measured with various methods. One method relies on the availability of an assay which measures the activity of the particular protein being translated. An example of an assay for measuring protein activity is a luciferase assay, or chloramphenicol acetyltransferase assay. These assays measure the amount of functionally active protein produced from the translation reaction. Activity assays will not measure full length protein that is inactive due to improper protein folding or lack of other post-translational modifications necessary for protein activity. Alternatively, specific proteins might be detected according to their size by capillary electrophoresis.

As used herein, the term "nucleic acid construct" refers to a nucleic acid that serves as substrate for transcribing at least one RNA that can be translated into a polypeptide or protein. The construct includes nucleic acids composed of DNA or RNA. Suitable sources of DNA for use a nucleic acid for an expression template including genomic DNA, cDNA and RNA that can be converted into cDNA. Genomic DNA, cDNA and RNA can be from any biological source, such as a tissue sample, a biopsy, a swab, sputum, a blood sample, a fecal sample, a urine sample, a scraping, among others. The genomic DNA, cDNA and RNA can be from host cell or virus origins and from any species, including extant and extinct organisms.

Another method of measuring the amount of protein produced in coupled *in vitro* transcription and translation reactions is to perform the reactions using a known quantity of radiolabeled amino acid such as ³⁵S-methionine, ³H-leucine, or ¹⁴C-leucine, and subsequently measure the amount of radiolabeled amino acid incorporated into the newly translated protein. Incorporation assays will measure the amount of radiolabeled amino acids in all proteins produced in the reaction, including truncated protein products. The radiolabeled protein may be further separated on a protein gel, and by autoradiography confirmed that the product is the proper size, and that secondary protein products have not been produced.

In **a fifth aspect,** the invention pertains to a method for screening a candidate compound for an effect on a target molecule, the method comprising:
(a) contacting a candidate compound and a target molecule in a cell-free system according to the invention;
(b) determining the amount or level of effected target molecule over time compared to a control or reference,

wherein the effect on the target molecule is selected from the group consisting of a reversible post translational modification, ubiquitin-proteasome dependent protein degradation, lysosome-dependent protein degradation, inhibition of the ubiquitin-proteasome dependent protein degradation, and RNA degradation,
preferably ubiquitin-proteasome dependent protein degradation, wherein an increased or decreased protein stability or protein degradation compared to the control or reference indicates that the candidate compound is a compound having an effect on ubiquitin-proteasome dependent protein degradation of the target molecule.

The term "contacting" in the present invention means any interaction between the potentially candidate compound and a target molecule.

As used herein, the term "target molecule" refers to a nucleic acid such as RNA or a protein, which is a target for modification or degradation in the cell-free protein synthesis system.

In the context of the herein disclosed invention the term "protein stability" is generally used in a structural context, i.e. relating to the structural integrity and half-life of a protein, or in a functional context, i.e. relating to a protein's ability to retain its function and/or activity over time. Protein stability can be influenced by proteolytic cleavage, loss of structural integrity of the three-dimensional folding, general physiological protein turn-over. Protein stability can be measured by a wide range of processes known to the skilled artisan and include, without being limited to any particular method, immunological methods using antibodies binding to three dimensional epitopes, pulse-chase methods such as cycloheximide chase and functional assays measuring time-dependent decline of enzyme activity.

Preferred is the method according to the present invention, wherein the target molecule is contacted with the cell-free system by expression of a target protein by the cell-free system, and wherein the cell-free system is modified by:
**(a)** adding a nucleic acid construct suitable for expressing a target protein to the cell-free protein synthesis system according to the invention or optionally adding the target molecule such as a target protein;
**(b)** optionally adding a E3 ubiquitin ligase, a E2 ubiquitin-conjugating enzyme, and/or a E1 ubiquitin-activating enzyme;
**(c)** adding the candidate compound suspected of having an effect on the target molecule,
**(d)** incubating to allow for target protein expression and modification/ degradation,
**(e)** measuring the effect on the target molecule, preferably compared to a control reaction in a cell-free protein synthesis system without a candidate compound.

Preferred is the method according to the present invention, wherein the candidate compound suspected of having an effect on the target molecule comprises PROteolysis TArgeting Chimeras (PROTACs), peptide PROTACs, molecular glues, PhotoPROTAC, Homo-PROTAC, Phosphatase Recruiting Chimera (PhoRC) Phosphorylation Targeting Chimera (PhosTAC), Phosphorylation-Inducing Chimeric Small Molecule (PHIC), Deubiquitinase-Targeting Chimera (DubTAC), Acetylation Tagging System (AceTAG), Lysosome-Targeting Chimera (LYTAC), Ribonuclease Targeting Chimera (RIBOTAC), and inhibitor of the ubiquitin-proteasome dependent protein degradation.

According to the present invention, the term "candidate compound" refers to a small molecule with a molecular weight of below 2000 Da, preferably of below 1000 Da, optimally in the range of between 600 and 200 Da. Most preferred is a molecular weight of below 500 Da. Additionally, the term refers to a peptide.

According to the present invention, the term "inhibitor of the ubiquitin-proteasome dependent protein degradation" refers to inhibitors of the 20S proteolytic core of the proteasome and compounds that have the ability to interact with the N-terminal threonine residues in the catalytic proteasome subunits that are responsible for the nucleophilic attacks involved in the cleavage of peptide bonds. These inhibitors are well-known in the art and have been described for example in Manasanch and Orlowski, 2017 (47).

Preferred is the method according to the present invention, wherein the measuring of the effect on the target molecule from step (e) is performed through a method selected from the group consisting of western blot, capillary immunoassay, fluorescence- or luminescence-based reporter assays, mass spectrometry, ALPHA assay, and ELISA assay.

In **a sixth aspect,** the invention pertains to a use of the cell-free protein synthesis system according to the invention for protein expression and/or for investigating ubiquitin-proteasome dependent protein degradation, inhibition of the ubiquitin-proteasome dependent protein degradation, protein stabilization, protein phosphorylation, protein dephosphorylation, protein acetylation, lysosome-dependent protein degradation, or RNA degradation.

In **a seventh aspect,** the invention pertains to a cell-free system kit, wherein the kit comprises:
(a) an aqueous cellular lysate according to the invention;
(b) optionally, additional compounds such as nucleotide triphosphates (NTPs), amino acids, magnesium acetate, GADD34, K3L, caspase inhibitor, DUB inhibitor, creatine phosphate, potassium acetate, DTT, creatine kinase, spermidine, m⁷GpppG cap analogue, T7 polymerase;
(c) and, optionally, instructions directing a user to combine the aqueous cellular lysate and the compounds from the kit with a nucleic acid construct encoding a target protein or a peptide PROTAC.
wherein the aqueous cellular lysate and the compounds are disposed in one or more separate containers.

In **an eighth aspect,** the invention pertains to a cell-free system, comprising the following components:
(a) magnesium acetate, preferably in a concentration range between 1 mM and 3 mM;
(b) an inhibitor of phosphorylation of eIF2α;
(c) an agonist of dephosphorylation of eIF2α; and/or
(d) a caspase inhibitor;
wherein the cell-free system is a prepared from a cell lysate and comprises a functional transcription/translation capability for protein expression and/or a functional ubiquitin-proteasome system for protein degradation.

Preferred is the method according to the present invention, wherein the cell-free system is prepared by a method according to the invention.

Therefore, the human cell-free *in vitro* transcription-translation (IVTT) system with active UPS is advantageous for high-throughput screening of new compounds for targeted protein degradation. For the validation of the compounds in terms of time, cost and degradation efficiency are used commercial PROTACs and molecular glues.

The new features of the human cell-free *in vitro* transcription-translation (IVTT) system open up completely new possibilities for research-based pharmaceutical companies to quickly and specifically identify new candidate molecules for the treatment of diseases for which there are currently no drugs.

The cell-free protein synthesis system can be adapted to other potential applications such as other proximity-inducing substances, translation inhibitors, RNA editing and RNA silencing.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

In view of the above, it will be appreciated that the present invention also relates to the following itemised embodiments:
Item 1. A method for the preparation of a cell lysate for use in, or as, a cell-free system, the method comprises the steps of:
   (a) cultivating and harvesting human cells;
   (b) optionally, washing said cultured human cells with a washing buffer;
   (c) admixing the cells with a lysis buffer, wherein the lysis buffer comprises one or more components selected from the group consisting of:
      (i) magnesium acetate, preferably in a concentration range between 1 mM and 3 mM;
      (ii) an inhibitor of phosphorylation of eIF2α;
      (iii) an agonist of dephosphorylation of eIF2α; and
      (iv) a caspase inhibitor;
   (d) lysing the cells by incubating the cells in the admixture of step (c), and preferably applying physical stress to the cells, to obtain a crude cell lysate;
   (e) purifying the crude cell lysate, such as by removing cellular debris from the crude cell lysate, for example by centrifugation and/or filtration and thereby obtaining an aqueous cell lysate;
   (f) optionally, cryoprotecting the aqueous cell lysate obtained in (e), for example by admixing the aqueous cell lysate with trehalose, preferably in a concentration range between 1.25 and 5 % (w/v).
Item 2. The method of item 1, wherein the human cells are human embryonic kidney (HEK) cells, preferably HEK293T cells.
Item 3. The method of item 1 or 2, wherein the inhibitor of phosphorylation of eIF2α is a pseudosubstrate for an eIF2α kinase, preferably K3L; optionally wherein the K3L is used in a concentration range between 0.01 mg/mL and 0.03 mg/mL.
Item 4. The method of any one of items 1 to 3, wherein the agonist of dephosphorylation of eIF2α is growth arrest and DNA damage-inducible protein (GADD34), preferably in a concentration range between 0.01 mg/mL and 0.02 mg/mL.
Item 5. The method of any one of items 1 to 4, wherein the lysis buffer comprises at least component (iv), preferably (i) and (iv), more preferably (i), (ii) and (iv) and most preferably components (i) to (iv).
Item 6. The method of any one of items 1 to 5, wherein the caspase inhibitor comprises a broad spectrum caspase inhibitor, preferably a caspase-1 inhibitor, a caspase-3 inhibitor, a caspase-7 inhibitor, a caspase-9 inhibitor, and/or a caspase-12 inhibitor; more preferably a caspase-3 inhibitor, a caspase-6 inhibitor and/or a caspase-7 inhibitor, for example wherein the caspase-1 inhibitor is selected from the group consisting of YVAD-CHO, VX-740 and VX-765; and/or wherein the caspase-3 inhibitor is selected from the group consisting of Ac-DEVD-CHO and Z-DEVD-FMK; and/or wherein the caspase-7 inhibitor is Q-VD-OPh; and/or wherein the caspase-9 inhibitor is Z-LEHD-FMK; and/or wherein the caspase-12 inhibitor is Z-ATAD-FMK.
Item 7. The method of item 1, wherein the physical force is selected from the group consisting of pressure, freezing and thawing, sonication, impact forces and shearing forces, such as sieving.
Item 8. The method of item 7, wherein the applying physical force comprises sieving the cells to mesh/sieve and/or passing the cells through a tip of a needle cannula.
Item 9. The method of item 8, wherein the mesh/sieve comprises an average pore size of 100 nm to 50 µm, preferably 500 nm to 25 µm, most preferably 1 to 10 µm (such as about 6 µm).
Item 10. The method of item 8, wherein the needle cannula has a diameter of less than 2 mm, preferably of less than 1 mm, more preferably of less than 0.5 mm.
Item 11. The method of any one of items 7 to 10, wherein the method comprises passing the cells through the mesh/sieve and/or needle cannula at least once, preferably at least twice, more preferably for 5 times or more (such as about 7 times through a mesh/sieve and/or about 10 times for a needle cannula).
Item 12. The method of any one of items 1 to 11, wherein the lysis buffer further comprises ATP, preferably in a concentration range between 1.5 mM and 2.5 mM.
Item 13. The method of any one of items 1 to 12, wherein the lysis buffer further comprises glycerol, preferably in a concentration range between 1.5 and 2.5 % w/v.
Item 14. A method of preparing a cell-free protein synthesis system, the method comprises a step of providing the aqueous cellular lysate of any one of items 1 to 13, and adding to the aqueous cellular lysate at least:
   (a) nucleotide triphosphates (NTPs);
   (b) an amino acid mixture;
   (c) one or more of the following components (i) to (v):
      (i) magnesium acetate, preferably in a concentration range between 1 mM and 3 mM;
      (ii) an inhibitor of phosphorylation of eIF2α;
      (iii) an agonist of dephosphorylation of eIF2α;
      (iv) a caspase inhibitor;
      (v) a deubiquitinases (DUB) inhibitor, preferably DUB inhibitor PR619; and/or
   (d) a nucleic acid construct encoding a protein to be expressed with the cell-free protein synthesis system.
Item 15. The method of item 14, wherein the nucleic acid construct (d) is added to a final concentration in the cell-free protein synthesis system of 12 nM to 28 nM, preferably 14 nM to 26 nM, more preferably 16 nM to 24 nM, even more preferably 18 nM to 22 nM.
Item 16. The method of item 14 or 15, further comprising a step of adding creatine phosphate, preferably in a concentration range of 15 mM to 45 mM, more preferably 20 mM to 40 mM, even more preferably 25 mM to 35 mM.
Item 17. The method of any one of items 14 to 16, further comprising a step of adding one or more of:
   (a) potassium acetate;
   (b) a reducing agent, preferably dithiothreitol (DTT) and/or tris(2-carboxyethyl)phosphine (TCEP);
   (c) creatine kinase;
   (d) spermidine;
   (e) m⁷GpppG cap analogue; and/or
   (f) T7 polymerase.
Item 18. A cell-free system obtained by, or obtainable by, performing a method of any one of items 1 to 17.
Item 19. The cell-free system of item 18, wherein the system is a cell-free protein synthesis system and comprises:
   (a) an aqueous cellular lysate prepared with a method of any one of items 1 to 13;
   (b) nucleotide triphosphates (NTPs);
   (c) an amino acid mixture;
   (d) an active ubiquitin-proteasome system;
   (e) One or more of the following components (i) to (v):
      (i) magnesium acetate, preferably in a concentration range between 1 mM and 3 mM;
      (ii) an inhibitor of phosphorylation of eIF2α;
      (iii) an agonist of dephosphorylation of eIF2α;
      (iv) a caspase inhibitor;
      (v) a deubiquitinases (DUB) inhibitor, preferably DUB inhibitor PR619 and/or
   (f) a nucleic acid construct encoding a protein to be expressed with the cell-free protein synthesis system.
Item 20. A method for expressing a protein, the method comprising the steps of:
   (a) adding a nucleic acid construct suitable for expressing the protein with the cell-free protein synthesis system of item 18 or 19.
   (b) isolating the protein encoded by the nucleic acid construct.
Item 21. A method for screening a candidate compound for an effect on a target molecule, the method comprising:
   (a) contacting a candidate compound and a target molecule in a cell-free system of any one of the preceding items;
   (b) determining the amount or level of effected target molecule over time compared to a control or reference,

   wherein the effect on the target molecule is selected from the group consisting of a reversible post translational modification, ubiquitin-proteasome dependent protein degradation, lysosome-dependent protein degradation, inhibition of the ubiquitin-proteasome dependent protein degradation, and RNA degradation,
   preferably ubiquitin-proteasome dependent protein degradation, wherein an increased or decreased protein stability or protein degradation compared to the control or reference indicates that the candidate compound is a compound having an effect on ubiquitin-proteasome dependent protein degradation of the target molecule.
Item 22. The method of item 21, wherein the target molecule is contacted with the cell-free system by expression of a target protein by the cell-free system, and wherein the cell-free system is modified by:
   (a) adding a nucleic acid construct suitable for expressing a target protein to the cell-free protein synthesis system of item 18 or 19 or optionally adding the target molecule such as a target protein;
   (b) optionally adding a E3 ubiquitin ligase, a E2 ubiquitin-conjugating enzyme, and/or a E1 ubiquitin-activating enzyme;
   (c) adding the candidate compound suspected of having an effect on the target molecule,
   (d) incubating to allow for target protein expression and modification/ degradation,
   (e) measuring the effect on the target molecule, preferably compared to a control reaction in a cell-free protein synthesis system without a candidate compound.
Item 23. The method of item 21 or 22, wherein the candidate compound suspected of having an effect on the target molecule comprises PROteolysis TArgeting Chimeras (PROTACs), peptide PROTACs, molecular glues, PhotoPROTAC, Homo-PROTAC, Phosphatase Recruiting Chimera (PhoRC) Phosphorylation Targeting Chimera (PhosTAC), Phosphorylation-Inducing Chimeric Small Molecule (PHIC), Deubiquitinase-Targeting Chimera (DubTAC), Acetylation Tagging System (AceTAG), Lysosome-Targeting Chimera (LYTAC), Ribonuclease Targeting Chimera (RIBOTAC), and inhibitor of the ubiquitin-proteasome dependent protein degradation.
Item 24. The method of item 21 to 23, wherein the measuring of the effect on the target molecule from step (e) is performed through a method selected from the group consisting of western blot, capillary immunoassay, fluorescence- or luminescence-based reporter assays, mass spectrometry, ALPHA assay, and ELISA assay.
Item 25. A use of the cell-free protein synthesis system of item 18 or 19 for protein expression and/or for investigating ubiquitin-proteasome dependent protein degradation, inhibition of the ubiquitin-proteasome dependent protein degradation, protein stabilization, protein phosphorylation, protein dephosphorylation, protein acetylation, lysosome-dependent protein degradation, or RNA degradation.
Item 26. A cell-free system kit, wherein the kit comprises:
   (a) an aqueous cellular lysate of any one of items 1 to 13;
   (b) optionally, additional compounds such as nucleotide triphosphates (NTPs), amino acids, magnesium acetate, GADD34, K3L, caspase inhibitor, DUB inhibitor, creatine phosphate, potassium acetate, DTT, creatine kinase, spermidine, m⁷GpppG cap analogue, T7 polymerase;
   (c) and, optionally, instructions directing a user to combine the aqueous cellular lysate and the compounds from the kit with a nucleic acid construct encoding a target protein or a peptide PROTAC.
   wherein the aqueous cellular lysate and the compounds are disposed in one or more separate containers.
Item 27. A cell-free system, comprising the following components:
   (a) magnesium acetate, preferably in a concentration range between 1 mM and 3 mM;
   (b) an inhibitor of phosphorylation of eIF2α;
   (c) an agonist of dephosphorylation of eIF2α; and/or
   (d) a caspase inhibitor;
   wherein the cell-free system is prepared from a cell lysate and comprises a functional transcription/translation capability for protein expression and/or a functional ubiquitin-proteasome system for protein degradation.
Item 28. The cell-free system of item 27, wherein the cell-free system is prepared by a method of any one of items 1 to 13.

### BRIEF DESCRIPTION OF THE FIGURES

The figures show:

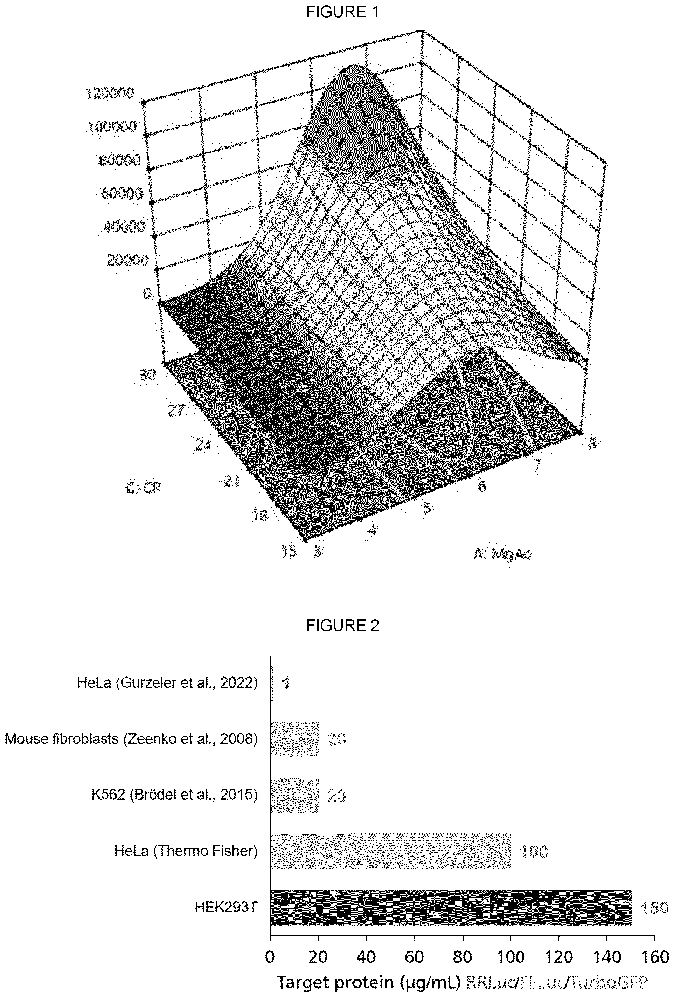

**Figure 1****:** shows a response surface and contour diagram for the TurboGFP yield in the coupled HEK293T IVTT system. The effect of the two factors magnesium acetate (MgAc) and creatine phosphate (CP) on the yield is shown, while the other two factors plasmid and spermidine were kept at the optimum concentrations determined.
**Figure 2****:** shows a yield comparison of different available human cell-free IVTT systems in batch mode.

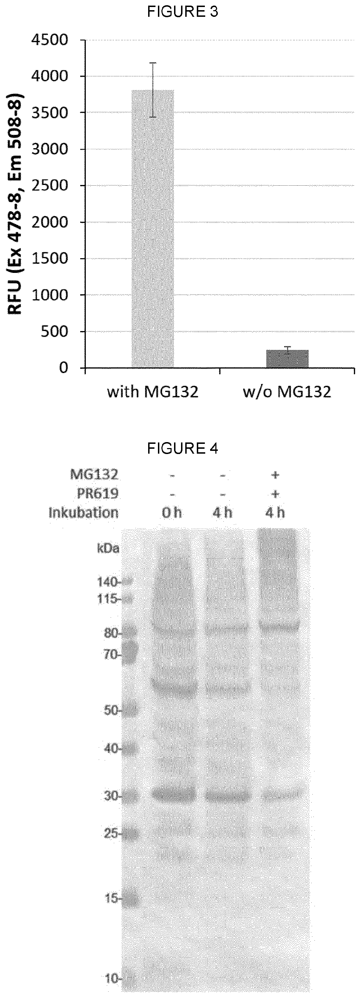

**Figure 3****:** shows the proteasome activity in HEK293T cell-free IVTT system. The expression of a fusion protein consisting of four ubiquitins, GFP and a disordered stretch of 35 amino acids derived from *Saccharomyces cerevisiae* cytochrome b2 in the coupled IVTT system without and with the proteasome inhibitor MG132 was analysed. **Lysate 3** (Table 1) and the optimised concentrations of the IVTT components were used (Table 5). The nucleic acid expression construct was 20 nM of the plasmid pCFE_Ub4-cP8GFP-35-His. The reactions were carried out at 15 µL scale in a 384 well plate at 33 °C and 500 rpm for 4 h. Prior to fluorescence measurement, samples were diluted 1:10 in 20 mM MOPS-NaOH pH 7.2. The data represent the mean values and standard deviations of three independent reactions.
**Figure 4****:** shows the verification of ubiquitination in the HEK293T cell-free IVTT system. The ubiquitination of proteins in the HEK293T cell-free IVTT system was shown by western blot analysis using a ubiquitin-specific HRP-coupled antibody. Lysate 2 (Table 1) and the optimised concentrations of the components without DTT were used (Table 5). The proteasome inhibitor MG132 and the deubiquitinase inhibitor PR619 were added to one preparation. One reaction was stopped immediately after preparation by freezing at -80 °C. The other reactions were incubated at 15 µL scale in a 384 well plate at 33 °C and 500 rpm for 4 h.

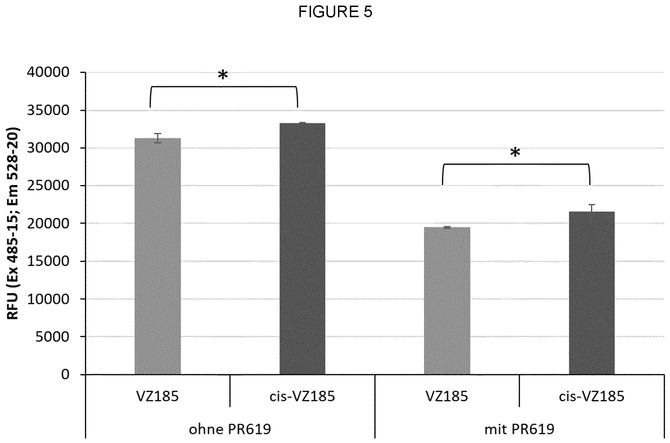

**Figure 5****:** shows PROTAC-mediated degradation of a target protein in the HEK293T cell-free IVTT system. The degradation of the bromodomain-containing protein 7 (BRD7), which is mediated by the PROTAC VZ185, with and without the DUB inhibitor PR619 is shown. The inactive PROTAC cis-VZ185, which cannot bind to the E3 ligase VHL, served as a control. **Lysate 3** (Table 1) and the optimised concentrations of the components without DTT were used (Table 5). The reactions were carried out at 15 µL scale in a 384 well plate at 33 °C and 500 rpm for 4 h. BRD7 was quantified using a split-GFP system. The data represent the means and standard deviations of three independent reactions each.

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Example 1: Development and optimization of a HEK293T cell-free IVTT system

In the context of the present invention, the inventors conducted a research study for suitable protocols for lysate preparation from human cells. The research study revealed protocols from Mikami et al. (2006a) (27), Wakiyama et al. (2007) (28), Mikami et al. (2008) (29), Rakotondrafara and Hentze (2011) (30), Bradrick et al. (2013) (31), Brödel et al. (2015) (32), Karousis et al. (2020) (33) and Gurzeler et al. (2022) (34).

The protocol from Brödel et al. (2015) (32) was developed for human K562 and CHO cells and was used as a starting point for the preparation of HEK293T lysates. Numerous HEK293T lysates were prepared testing various lysis buffers and different cell disruption methods. Moreover, the lysate clarification step and the cryoprotection of the lysate were optimized.

**Table 1: Overview of lysates.**

| | **Lysate 1** | **Lysate 2** | **Lysate 3** | **Lysate 4** |
|---|---|---|---|---|
| **Cell density** | 7.6*10⁶ cells per mL | 7.1*10⁶ cells per mL | 4.1*10⁶ cells per mL | 3.3*10⁶ cells per mL |
| **Wash buffer** | 40 mM HEPES-KOH, pH 7,5 | | | |
| | 100 mM NaAc | | | |
| | 4 mM DTT | | | |
| **Lysis buffer** | Wash buffer | Wash buffer | Wash buffer | |
| | | | 5 mM MgAc | |
| | 2 mM MgAc | 2 mM MgAc | 0.02 mg/mL GADD34 | |
| | 0,02 mg/mL GADD34 | 0,02 mg/mL GADD34 | 0,0266 mg/mL K3L | |
| | 0,0266 mg/mL K3L | 0,0266 mg/mL K3L | 100 µM Ac-DEVD-CHO | |
| | | 100 µM Ac-DEVD-CHO | 2 mM ATP | |
| | | | 2 % (v/v) Glycerol | |
| **Lysis method** | 6 µm cell sieve (7x) | 27G ½-inch needle (10x) | 6 µm cell sieve (3x) | |
| **Clarification step** | 2x10.000xg for 5 min | 10.000xg for 5 min | 700xg for 5 min | |
| **Cryoprotectant** | - | Trehalose (2,5 % (w/v)) | | |
| **Freezing** | N₂ | Directly at -80 °C | | |
| **Protein concentration** | 27 mg/mL | 20 mg/mL | 9 mg/mL | 14 mg/mL |
| **TurboGFP yield** | 33 µg/mL | 50 µg/mL | 61 µg/mL | 151 µg/mL |

Table 1 shows an overview of the lysates used for optimization of the cell-free reaction conditions by a design of experiments (DoE)-based approach. These lysates were used for a HEK293T cell-free *in vitro* transcription-translation (IVTT) system and the proteasome activity, protein ubiquitination and PROTAC-mediated target protein degradation in this system were tested.

**Lysate 1** was prepared according to Brödel et al. (2015) with several modifications. In contrast to Brödel et al. (2015) 2 mM magnesium acetate and the accessory proteins GADD34 (0.02 mg/mL) and K3L (0.0266 mg/mL) were added to the lysis buffer. Magnesium plays a major role in many steps of protein synthesis, for example (i) the aminoacetylation of tRNA, (ii) formation of the ternary complex between aminoacylated initiator methionine tRNA, GTP, and initiation factor 2, and (iii) ribosomal complex maintenance. GADD34 and K3L can inhibit the phosphorylation of eIF2α, which leads to a considerable inhibition of protein synthesis (35). K3L acts as a pseudosubstrate for eIF2α kinases and prevents phosphorylation of eIF2α (36).

GADD34 recruits a phosphatase PP1c to elF2 to dephosphorylate eIF2α (37). To keep the phosphorylation of eIF2α caused by cell stress during the disruption procedure as low as possible, GADD34 and K3L were already added to the lysis buffer and not only to the cell-free reactions. GADD34 and K3L were produced in a tobacco BY-2 cell-free system and were purified thereof by Strep-tag affinity chromatography. The purified proteins were stored in 20 mM HEPES-KOH pH 7.5, 100 mM KCI and 5 mM DTT at -20 °C.

The HEK293T cells (HEK293T-CRL-3216TM, American Type Culture Collection, ATCC, Manassas, USA) were cultivated as a suspension in serum-free FreeStyle 293 expression medium (#12338018, Thermo Fisher, Waltham, USA) at 200 mL scale in 1000 mL shake flaks with 2 µm vent cap (#431147, Corning, Glendale, USA) at 37 °C, 180 rpm and 5 % CO₂. The cells were harvested at a cell density of 7.6*10⁶ cells per mL. After washing cells two times with washing buffer (according to Brödel et al. (2015) (32), 40 mM HEPES-KOH pH 7,5, 100 mM sodium acetate, 4 mM DTT), the cells were resuspended in one pellet volume of lysis buffer (40 mM HEPES-KOH pH 7,5, 100 mM sodium acetate, 4 mM DTT, 2 mM magnesium acetate, 0.02 mg/mL GADD34, 0.0266 mg/mL K3L). Afterwards the cells were disrupted by seven passages through a 6 µm cell sieve, which was assembled to a 50 mL syringe. Nuclei and non-disrupted cells were removed by centrifugation at 10,000 xg for 10 min at 4 °C. The supernatant was flash-frozen in liquid nitrogen and was then frozen in 100 µL aliquots at -80 °C.

The obtained lysate was tested in coupled *in vitro* transcription-translation (IVTT) reactions in triplicate using reaction conditions according to Brödel et al. (2015) (32) with some modifications (Table 2). The magnesium acetate concentration was increased from 3.4 mM to 4.8 mM. Moreover, GADD34 and K3L were added to a final concentration of 0,01 mg/mL and 0,0133 mg/mL, respectively, as described by Mikami et al. (2006a) (35). Using plasmid pCFE_GFP, a commercial vector for the human cell-free system from Thermo Fisher (Waltham, USA), as nucleic acid expression construct, reactions were carried out at 15 µL scale in 384 well plates at 33°C and 500 rpm for 3 h. **Lysate 1** produced 33 µg/mL TurboGFP using the initial reaction conditions.

**Table 2: Initial concentrations of the components for the coupled HEK293T IVTT system.**

| **Component** | **Concentration** |
|---|---|
| **Magnesium acetate** | 4.8 mM |
| **Potassium acetate** | 120 mM |
| **Plasmid** | 15 nM |
| **ATP** | 1.75 mM |
| **GTP/CTP/UTP** | 0.3 mM |
| **DTT** | 2.5 mM |
| **Creatine phosphate** | 20 mM |
| **Creatine kinase** | 0.1 mg/mL |
| **Amino acids** | 0.1 mM |
| **Spermidine** | 0.25 mM |
| **m⁷GpppG cap analogue** | 0.3 mM |
| **T7 polymerase** | 0.011 mg/mL |
| **GADD34** | 0.01 mg/mL |
| **K3L** | 0.0133 mg/mL |
| **Lysate** | 40 % (v/v) |

Many of the components in IVTT reactions are interdependent (38). Therefore, in the next step, a statistical design of experiments (DoE) was used to further optimize the system. The four factors selected for testing were magnesium acetate, plasmid, creatine phosphate and spermidine. These were analyzed for optimal concentrations using a quadratic IV optimal design with 32 approaches. Table 3 shows the concentration ranges for each of the factors analyzed. The other components remained unchanged (Table 2). **Lysate 1** was used and the plasmid pCFE_GFP served as a nucleic acid expression construct. The IVTT reactions were incubated at 15 µL scale in 384 well plates at 33 °C and 500 rpm for 4 hours.

**Table 3: Concentration ranges of the factors analysed in the DoE.**

| **Numercal factor** | **Factor name** | **Range** |
|---|---|---|
| **A** | Magnesium acetate | 3-8 mM |
| **B** | Plasmid | 9/15/20 nM |
| **C** | Creatine phosphate | 15/20/30 mM |
| **D** | Spermidine | 0/0.25/0.5 mM |

The two best DoE approaches achieved 53 µg/mL TurboGFP. Response surface models were used to predict the factor values required for optimal TurboGFP yield (39). A quadratic model was fitted to the experimental data. All non-significant terms (P>0.05 by ANOVA) were removed. The ANOVA table showed that the model was significant (P<0.0001). The fit of the model was also confirmed by the multiple R² and adjusted R² values (0.938 and 0.920, respectively) (Table 4). A strong interaction between magnesium acetate and creatine phosphate was observed (Figure 1). As expected, higher amounts of creatine phosphate required a higher concentration of magnesium acetate. In addition, higher plasmid concentrations led to higher TurboGFP yields. Spermidine, on the other hand, had no major influence.

**Table 4: ANOVA table for the optimization of the IVTT reaction components.**

| **Source** | | **Sum of Squares** | | **df** | **Mean Square** | **F-value** | **p-value** |
|---|---|---|---|---|---|---|---|
| **Model** | | | 102.12 | 7 | 14.59 | 52.06 | < 0.0001 significant |
| A-Magnesium acetate | | | 49.96 | 1 | 49.96 | 178.25 | < 0.0001 |
| B-Plasmid | | | 2.76 | 1 | 2.76 | 9.86 | 0.0044 |
| C-Creatine phospthate | | | 1.74 | 1 | 1.74 | 6.21 | 0.0200 |
| D-Spermidine | | | 0.0001 | 1 | 0.0001 | 0.0003 | 0.9862 |
| AC | | | 7.85 | 1 | 7.85 | 28.02 | < 0.0001 |
| AD | | | 4.12 | 1 | 4.12 | 14.69 | 0.0008 |
| A² | | | 46.04 | 1 | 46.04 | 164.29 | < 0.0001 |
| **Residual** | | | 6.73 | 24 | 0.2803 | | |
| Lack of Fit | | | 6.37 | 20 | 0.3185 | 3.57 | 0.1127 not significant |
| Pure Error | | | 0.3569 | 4 | 0.0892 | | |
| **Cor Total** | | | 108.85 | 31 | | | |
| Std. Dev. | 0.5294 | R² | 0.9382 | | | | |
| Mean | 8.84 | Adjusted R² | 0.9202 | | | | |
| C.V. % | 5.99 | Predicted R² | 0.8949 | | | | |
| | | Adeq Precision | 24.7212 | | | | |

As a result of the experiment, the concentration of magnesium acetate was increased from 4.8 mM to 6.9 mM, creatine phosphate from 20 mM to 30 mM and plasmid from 15 nM to 20 nM for subsequent IVTT reactions. Spermidine remained unchanged at 0.25 mM. Table 5 shows the composition of the optimized IVTT reactions.

**Table 5: Optimized concentrations of the components for the coupled HEK293T IVTT system.**

| **Component** | **Concentration** |
|---|---|
| **Magnesium acetate** | 6.9 mM |
| **Potassium acetate** | 120 mM |
| **Plasmid** | 20 nM |
| **ATP** | 1.75 mM |
| **GTP/CTP/UTP** | 0.3 mM |
| **DTT** | 2.5 mM |
| **Creatine phosphate** | 30 mM |
| **Creatine kinase** | 0.1 mg/mL |
| **Amino acids** | 0.1 mM |
| **Spermidine** | 0.25 mM |
| **m⁷GpppG cap analogue** | 0.3 mM |
| **T7 polymerase** | 0.011 mg/mL |
| **GADD34** | 0.01 mg/mL |
| **K3L** | 0.0133 mg/mL |
| **Lysate** | 40 % (v/v) |

For **lysate 2** HEK293T cells were cultivated as described for lysate 1. The cells were harvested at a cell density of 7.1*10⁶ cells per mL. After washing cells two times with washing buffer (40 mM HEPES-KOH pH 7.5, 100 mM sodium acetate, 4 mM DTT), cells were resuspended in one pellet volume of lysis buffer (40 mM HEPES-KOH pH 7.5, 100 mM sodium acetate, 4 mM DTT, 2 mM magnesium acetate, 0.02 mg/mL GADD34, 0.0266 mg/mL K3L, 100 µM Ac-DEVD-CHO (#S7901, Absource Diagnostics GmbH, München, Germany)). The caspase inhibitor Ac-DEVD-CHO was added to block caspase signaling cascades as they can induce apoptosis, which can lead to phosphorylation of eIF2α (40). In addition, caspase activation can cause the cleavage of three specific subunits of the 19S regulatory complex of the proteasome and thus the inhibition of proteasomal degradation (41).

However, the proteasome activity should be retained in the lysate. Afterwards the cells were disrupted by ten passages through a 27G ½ inch needle, which was assembled to a 1 mL syringe. Nuclei and non-disrupted cells were removed by centrifugation at 10,000 xg for 5 min at 4 °C. The supernatant was supplemented with 2.5 % (w/v) trehalose as a cryoprotectant and was then frozen in 100 µL aliquots at -80 °C.

To test the translational activity of the lysate, coupled IVTT reactions were set up in triplicate. The optimized concentrations of the IVTT components were used as obtained from the DoE-based experiment (Table 5). However, the reactions also contained 20 µM of the caspase inhibitor Ac-DEVD-CHO coming from the lysis buffer used during lysate preparation. The plasmid pCFE_GFP served as a nucleic acid expression construct. The reactions were incubated at 15 µL scale in 384 well plates at 33 °C and 500 rpm for 4 h. **Lysate 2** produced 50 µg/mL TurboGFP.

For **lysate 3** HEK293T cells were cultivated as described for **lysate 1 and 2,** but the cells were harvested at a lower cell density of 4.1*10⁶ cells per mL. After washing cells two times with washing buffer (40 mM HEPES-KOH pH 7.5, 100 mM sodium acetate, 4 mM DTT), cells were resuspended in one pellet volume of lysis buffer (40 mM HEPES-KOH pH 7.5, 100 mM sodium acetate, 4 mM DTT, 5 mM magnesium acetate, 0.02 mg/mL GADD34, 0.0266 mg/mL K3L, 100 µM Ac-DEVD-CHO, 2 mM ATP, 2 % (v/v) glycerol). ATP and glycerol are described in the literature in most buffers for the isolation of active proteasomes (42, 43). For the same reason, the magnesium acetate concentration in the lysis buffer was increased from 2 mM to 5 mM. Afterwards the cells were disrupted by three passages through a 6 µm cell sieve, which was assembled to a 50 mL syringe. Nuclei and non-disrupted cells were removed by centrifugation at 700 xg for 5 min at 4 °C. The centrifugation speed was reduced from 10,000 xg to 700 xg to keep organelles like mitochondria and microsomes, which are vesicles derived from the endoplasmic reticulum, in the lysate. The supernatant was supplemented with 2.5 % (w/v) trehalose as a cryoprotectant and was then frozen in 100 µL aliquots at -80 °C.

To test the translational activity of the lysate, coupled IVTT reactions were set up in triplicate. The optimized concentrations of the IVTT components were used (Table 5), except that DTT was omitted as a potentially ubiquitination-inhibiting component. Moreover, the reactions additionally contained 20 µM of the caspase inhibitor Ac-DEVD-CHO, 0.4 mM ATP and 0.4 % (v/v) glycerol coming from the lysis buffer used during lysate preparation. The plasmid pCFE_GFP served as a nucleic acid expression construct. The reactions were incubated at 15 µL scale in 384 well plates at 33 °C and 500 rpm for 5 h. **Lysate 3** produced 61 µg/mL TurboGFP with the reduced DTT concentration.

**Lysate 4** HEK293T cells were cultivated as described for the previous lysates, but the cells were harvested at an even lower cell density of 3.3*10⁶ cells per mL. The rest of the procedure was as described for **lysate 3.**

To test the translational activity of the lysate, coupled IVTT reactions were set up in triplicate. The optimized concentrations of the IVTT components were used (Table 5). However, the reactions additionally contained 20 µM of the caspase inhibitor Ac-DEVD-CHO, 0.4 mM ATP and 0.4 % (v/v) glycerol coming from the lysis buffer used during lysate preparation. The plasmid pCFE_GFP served as a nucleic acid expression construct. The reactions were incubated at 15 µL scale in 384 well plates at 33 °C and 500 rpm for 5 h. **Lysate 4** produced 151 µg/mL TurboGFP with 2.5 mM DTT, indicating a positive impact of DTT on the target protein yield.

In summary, cell-free reactions using HEK293T lysate yielded in approximately 150 µg expressed target protein, per mL in coupled transcription-translation reactions in batch mode and are therefore superior to other human cell-free expression systems (Figure 2).

### Example 2: Verification of proteasome activity in the HEK293T cell-free IVTT system.

The proteasome activity in the HEK293T cell-free IVTT system was analysed by expression of a fusion construct consisting of four ubiquitins, GFP and a disordered stretch of 35 amino acids derived from *Saccharomyces cerevisiae* cytochrome b2, which is efficiently degraded by purified proteasomes (45). Using **lysate 3** coupled IVTT reactions were set up in triplicate with the optimised concentrations of the components described in Table 5 without and with 10 µM proteasome inhibitor MG132 (#474791-1MG, Sigma-Aldrich). The proteasome inhibitor MG132 was dissolved in DMSO and the reactions without inhibitor contained the same concentration of DMSO. The nucleic acid expression construct was 20 nM of the plasmid pCFE_Ub4-cP8GFP-35-His. The reactions were incubated at 15 µL scale in 384 well plates at 33 °C and 500 rpm for 4 h.

Figure 3 shows the relative fluorescence values after 4 h and ten-fold dilution in 20 mM MOPS-NaOH pH 7.2. Significantly higher values were achieved with the proteasome inhibitor MG132 than without, which shows degradation of the fusion protein by the proteasome. The degradation rate was 94 %, indicating high proteasomal activity in the HEK293T cell-free IVTT system.

### Example 3: Verification of ubiquitination in the HEK293T cell-free IVTT system.

The ubiquitination of proteins in the HEK293T cell-free IVTT system was demonstrated by western blot analysis. For this purpose, coupled IVTT reactions were carried out with **lysate 2.** The optimised concentrations of the IVTT components were used (Table 5). DTT, which potentially interferes with ubiquitination, was omitted. The degradation of ubiquitinylated proteins in the proteasome was blocked by 40 µM of the proteasome inhibitor MG132 (#474791-1MG, Sigma-Aldrich). Cleavage of ubiquitin residues by deubiquitinases (DUBs) was prevented by addition of 50 µM of the DUB inhibitor PR619 (#2645-32-1, Absource Diagnostics). A nucleic acid expression construct was not added. A reaction without inhibitors was stopped immediately after preparation by freezing at -80 °C. One reaction, each with and without inhibitors was incubated at 15 µL scale in 384 well plates at 33 °C and 500 rpm for 4 hours. Subsequently, 3 µL of each sample was loaded onto a NuPAGE^{™} 4-12 % Bis-Tris protein gel. After electrophoresis, the proteins were transferred to a nylon membrane and the ubiquitinylated proteins were detected using an HRP-coupled ubiquitin-specific antibody.

The sample before incubation (lane 1 in Figure 4) contained significantly more ubiquitinylated endogenous proteins than the sample without proteasome and DUB inhibitor after 4 h of incubation (lane 2 in Figure 4). This indicates a degradation of ubiquitinylated proteins by the proteasome or a cleavage of ubiquitin residues by DUBs. In contrast, the sample with inhibitors (lane 3 in Figure 4) shows significantly more bands in the range above 115 kDa, showing polyubiquitination of endogenous proteins in the HEK293T cell-free IVTT system. Monoubiquitin at around 9 kDa is only very faintly visible in all samples. The bands around 30 kDa and 60 kDa could be E1, E2 or E3 ligase. A decrease in band intensity indicates a transfer of the ubiquitin residue to other proteins. The HEK293T cell-free IVTT system is therefore capable of labelling proteins with ubiquitin.

### Example 4: PROTAC-mediated degradation of a target protein in the HEK293T cell-free IVTT system.

After the activity of the proteasome and ubiquitination in the HEK293T cell-free IVTT system had been demonstrated, the PROTAC-mediated degradation of a target protein was investigated. PROTAC VZ185 was chosen for this purpose. VZ185 is a VHL-based, potent and selective PROTAC that mediates the degradation of the bromodomain-containing proteins (BRD) 7 and 9. BRD7 and BRD9 play a role in chromatin remodeling and thus in the regulation of gene expression. Detection of the target protein or its degradation should be carried out using a split-GFP system (46). In this system, sfGFP is split asymmetrically between the β-strands 10 and 11 into a large (GFP1-10) and a small (GFP11) fragment. The two fragments do not fluoresce individually but can spontaneously interact with each other and form a functional GFP. By fusing the GFP11 fragment with a target protein and associating it with the GFP1-10 fragment, the relative concentration of the target protein can be determined.

A fusion construct of BRD7 and GFP11 was expressed in lysate 3. The plasmid pCFE_GFP11-BRD7 served as a nucleic acid expression construct and the optimised concentrations of the IVTT components were used (Table 5). DTT, which potentially interferes with ubiquitination, was omitted. In addition, 1 µM of the PROTAC VZ185 (#6936, Bio-Techne, Wiesbaden) or as a control 1 µM of the inactive PROTAC cis-VZ185 (#6939, Bio-Techne) was added. Cis-VZ185 is the (S)-hydroxydiastereoisomer of VZ185. Although it has a comparable bromodomain binding affinity, it can no longer bind and recruit the E3 ligase VHL and therefore does not induce the degradation of BRD7. For both PROTACs, 50 µM of the DUB inhibitor PR619 was also added to half of the reactions. The reactions were incubated at 15 µL scale in 384 well plates at 33 °C and 500 rpm for 4 hours. Subsequently, 10 µL of each sample was mixed with 40 µL of the GFP1-10 fragment (300 µg/mL in 100 mM Tris-HCl pH 8.0, 150 mM NaCl, 10 % (v/v) glycerol, 1 mM EDTA). This was previously produced with an N-terminal Strep-tag in a BY-2 cell-free system and purified thereof using Strep-tag affinity chromatography. After 40 h incubation at 4 °C, the fluorescence was measured (Figure 5). Both with and without DUB inhibitor, there were significantly lower fluorescence values (p < 0.05) for the reactions with the active PROTAC VZ185 compared to the control PROTAC cis-VZ185, which shows degradation of the target protein GFP11-BRD7. The difference with additional DUB inhibitor was slightly greater at 10 % than without at 6 %. PROTAC-mediated degradation of a target protein in the HEK293T cell-free IVTT system is therefore possible, but the efficiency should still be optimised.

### REFERENCES

References as cited:
1. Dang C. V.; Reddy E. P.; Shokat K. M.; Soucek L. Drugging the "undruggable" Cancer Targets. Nat. Rev. Cancer 17 (8): 502-508 (2017).
2. Spradlin J. N., Zhang E., Nomura D. K. Reimagining Druggability Using Chemoproteomic Platforms. Acc Chem Res., 6;54(7): 1801-1813 (2021). doi: 10.1021/acs.accounts.1c00065. Epub 2021 Mar 18. PMID: 33733731.
3. Sun X., Gao H., Yang Y., He M., Wu Y., Song Y., Tong Y, Rao Y. PROTACs: great opportunities for academia and industry. In: Signal transduction and targeted therapy 4: 64 (2019). DOI: 10.1038/s41392-019-0101-6.
4. Hughes S. J. & Ciulli A. Molecular recognition of ternary complexes: a new dimension in the structure-guided design of chemical degraders. Essays Biochem. 61: 505-516 (2017).
5. Toure M. & Crews C. M. Small-molecule PROTACS: new approaches to protein degradation. Angew. Chem. Int Ed. Engl. 55: 1966-1973 (2016).
6. Yang J. et al. Simple structural modifications converting a bona fide MDM2 PROTAC degrader into a molecular glue molecule: a cautionary tale in the design of PROTAC degraders. J. Med. Chem. 62: 9471-9487 (2019).
7. Banik S. M. et al. Lysosome targeting chimeras (LYTACs) for the degradation of secreted and membrane proteins. ChemRxiv: 1-68 (2019).
8. Reynders M. et al. PHOTACs enable optical control of protein degradation. ChemRxiv: 1-34 (2019).
9. Takahashi D. et al. AUTACs: cargo-specific degraders using selective autophagy. Mol. Cell 76: 797-810.e10 (2019).
10. Maniaci C. et al. Homo-PROTACs: bivalent small-molecule dimerizers of the VHL E3 ubiquitin ligase to induce self-degradation. Nat. Commun. 8: 830 (2017).
11. Chessum N.E.A., Sharp S.Y., Caldwell J.J., Pasqua A.E., Wilding B., Colombano G., Collins I., Ozer B., Richards M., Rowlands M., Stubbs M., Burke R., McAndrew P.C., Clarke P.A., Workman P., Cheeseman M.D., Jones K. Demonstrating In-Cell Target Engagement Using a Pirin Protein Degradation Probe (CCT367766). J Med Chem. 8; 61(3):918-933 (2018). doi: 10.1021/acs.jmedchem.7b01406. Epub 2018 Jan 5. PMID: 29240418; PMCID: PMC5815658.
12. Bai L., Zhou H., Xu R., Zhao Y, Chinnaswamy K., McEachern D., Chen J., Yang C.Y., Liu Z., Wang M., Liu L., Jiang H., Wen B., Kumar P., Meagher J.L., Sun D., Stuckey J.A., Wang S. A Potent and Selective Small-Molecule Degrader of STAT3 Achieves Complete Tumor Regression In Vivo. In: Cancer cell 36 (5), 498-511.e17. (2019). DOI: 10.1016/j.ccell.2019.10.002.
13. Lin J., Jin J., Shen Y., Zhang L., Gong G., Bian H., Chen H., Nagle D.G., Wu Y., Zhang W., Luan X. Emerging protein degradation strategies: expanding the scope to extracellular and membrane proteins. Theranostics. 13; 11(17):8337-8349 (2021). doi: 10.7150/thno.62686. PMID: 34373745; PMCID: PMC8344007.
14. Hughes S. J., Testa A., Thompson N., Churcher I. The rise and rise of protein degradation: Opportunities and challenges ahead. Drug Discov Today 26(12): 2889-2897 (2021). doi: 10.1016/j.drudis.2021.08.006. Epub 2021 Aug 20. PMID: 34419629.
15. Ma S., Ji J., Tong Y., Zhu Y, Dou J., Zhang X., Xu S., Zhu T., Xu X., You Q., Jiang Z. Non-small molecule PROTACs (NSM-PROTACs): Protein degradation kaleidoscope. Acta Pharm Sin B. 12(7): 2990-3005 (2022). doi: 10.1016/j.apsb.2022.02.022. Epub 2022 Feb 26. PMID: 35865099; PMCID: PMC9293674.
16. Pelay-Gimeno M., Glas A., Koch O., Grossmann T. N. Structure-based design of inhibitors of protein-protein interactions: mimicking peptide binding epitopes. Angew Chem Int Ed Engl;54: 8896e927 (2015).
17. Li Y., Liu M., Xie S. Harnessing Phage Display for the Discovery of Peptide-Based Drugs and Monoclonal Antibodies. Curr Med Chem. 28(40): 8267-8274 (2021). doi: 10.2174/0929867327666201111144353. PMID: 33176631.
18. Linciano S., Pluda S., Bacchin A., Angelini A. Molecular evolution of peptides by yeast surface display technology. Medchemcomm. 10;10(9): 1569-1580 (2019). doi: 10.1039/c9md00252a. PMID: 31803399; PMCID: PMC6836575.
19. Bondeson D. P., Smith B. E., Burslem G. M., Buhimschi A. D., Hines J., Jaime-Figueroa S., Wang J., Hamman B. D., Ishchenko A., Crews C. M. Lessons in PROTAC Design from Selective Degradation with a Promiscuous Warhead. Cell Chem Biol. 18;25(1): 78-87.e5 (2018). doi: 10.1016/j.chembiol.2017.09.010. Epub 2017 Nov 9. PMID: 29129718; PMCID: PMC5777153.
20. Dong G., Ding Y., He S., Sheng C. Molecular Glues for Targeted Protein Degradation: From Serendipity to Rational Discovery. J Med Chem. 12;64(15): 10606-10620 (2021). doi: 10.1021/acs.jmedchem.1c00895. Epub 2021 Jul 28. PMID: 34319094.
21. Buren T., Frahm M., Werber Y., Woods B., Rao D., Bancroft T., McCallister E. Profoundly Expanding The Target Space: A Primer On TPD. Ahead Of The Curve® Series (2022).
22. Kozicka Z. and Thomä N.H. Haven't got a glue: protein surface variation for the design of molecular glue degraders. Cell Chem Biol. 28: 1032-47 (2021). https://doi.org/10.1016/j.chembiol.2021. 04.009.
23. Weagel E. G., Foulks J. M., Siddiqui A., Warner S. L. Molecular glues: enhanced protein-protein interactions and cell proteome editing. Med Chem Res 31, 1068-1087 (2022). https://doi. org/10.1007/s00044-022-02882-2.
24. Roy M. J., Winkler S., Hughes S. J., Whitworth C., Galant M., Farnaby W., Rumpel K., Ciulli A. SPR-Measured Dissociation Kinetics of PROTAC Ternary 19 Complexes Influence Target Degradation Rate. ACS Chem Biol. 15;14(3): 361368 (2019). doi: 10.1021/acschembio.9b00092. Epub 2019 Feb 22. PMID: 30721025; PMCID: PMC6423499.
25. Scholes N. S., Mayor-Ruiz C., Winter G. E. Identification and selectivity profiling of small-molecule degraders via multi-omics approaches. Cell Chem Biol. 15;28(7): 1048-1060 (2021). doi: 10.1016/j.chembiol.2021.03.007. Epub 2021 Apr 2. PMID: 33811812.
26. Chini A. Application of yeast-two hybrid assay to chemical genomic screens: a high-throughput system to identify novel molecules modulating plant hormone receptor complexes. Methods Mol Biol. 1056:35-43 (2014). doi: 10.1007/978-1-62703-592-7_4. PMID: 24306860.
27. Mikami S., Masutani M., Sonenberg N., Yokoyama S., Imataka H. An efficient mammalian cell-free translation system supplemented with translation factors. Protein Expr Purif. 46(2): 348-57 (2006a). doi: 10.1016/j.pep.2005.09.021. Epub 2005 Oct 25. PMID: 16289705.
28. Wakiyama M., Takimoto K., Ohara O., Yokoyama S. Let-7 microRNA-mediated mRNA deadenylation and translational repression in a mammalian cell-free system. Genes Dev. 1;21(15): 1857-62 (2007). doi: 10.1101/gad.1566707. Erratum in: Genes Dev. 2007 Oct 1;21(19):2509. PMID: 17671087; PMCID: PMC1935024.
29. Mikami S., Kobayashi T., Masutani M., Yokoyama S., Imataka H. A human cell-derived in vitro coupled transcription/translation system optimized for production of recombinant proteins. Protein Expr Purif. 62(2):190-8 (2008). doi: 10.1016/j.pep.2008.09.002. Epub 2008 Sep 11. PMID: 18814849.
30. Rakotondrafara A. M., Hentze M. W. An efficient factor-depleted mammalian in vitro translation system. Nat Protoc. 6(5): 563-71. (2011). doi: 10.1038/nprot.2011.314. Epub 2011 Apr 7. PMID: 21527914.
31. Bradrick S. S., Nagyal S., Novatt H. A miRNA-responsive cell-free translation system facilitates isolation of hepatitis C virus miRNP complexes. RNA 19(8): 1159-69 (2013). doi: 10.1261/rna.038810.113. Epub 2013 Jun 21. PMID: 23793894; PMCID: PMC3708535.
32. Brödel A. K., Wüstenhagen D. A., Kubick S. Cell-free protein synthesis systems derived from cultured mammalian cells. Methods Mol Biol. 1261: 129-40 (2015). doi: 10.1007/978-1-4939-2230-7_7. PMID: 25502197.
33. Karousis E. D., Gurzeler L. A., Annibaldis G., Dreos R., Mühlemann O. Human NMD ensues independently of stable ribosome stalling. Nat Commun 11, 4134 (2020). https://doi.org/10.1038/s41467-020-17974-z.
34. Gurzeler L. A., Ziegelmüller J., Mühlemann O., Karousis E. D. Production of human translation-competent lysates using dual centrifugation. RNA Biol. 19(1): 78-88 (2022). doi: 10.1080/15476286.2021.2014695. Epub 2021 Dec 29. PMID: 34965175; PMCID: PMC8815625.
35. Mikami S., Kobayashi T., Yokoyama S., Imataka H. A hybridoma-based in vitro translation system that efficiently synthesizes glycoproteins. In: Journal of biotechnology: 65-78 (2006b). DOI: 10.1016/j.jbiotec.2006.06.018.
36. Carroll K., Elroy-Stein O., Moss B., Jagus R. Recombinant vaccinia virus K3L gene product prevents activation of double-stranded RNA-dependent, initiation factor 2 alpha-specific protein kinase. In: Journal of Biological Chemistry 268 (17): 12837-12842 (1993).
37. Novoa I., Zeng H., Harding H. P., Ron D. Feedback inhibition of the unfolded protein response by GADD34-mediated dephosphorylation of elF2alpha. In: The Journal of cell biology 153 (5): 1011-1022 (2001). DOI: 10.1083/jcb.153.5.1011.
38. Jewett M. C. and Swartz J. R. Mimicking the Escherichia coli cytoplasmic environment activates long-lived and efficient cell-free protein synthesis. In: Biotechnology and bioengineering 86 (1): 19-26 (2004). DOI: 10.1002/bit.20026.
39. Zhou Q., Su J., Jiang H., Huang X., Xu Y. Optimization of phenazine-1-carboxylic acid production by a gacA/qscR-inactivated Pseudomonas sp. M18GQ harboring pME6032Phz using response surface methodology. In: Applied microbiology and biotechnology 86 (6): 1761-1773 (2010). DOI: 10.1007/s00253-010-2464-z.
40. Saelens X., Kalai M., Vandenabeele P. Translation inhibition in apoptosis: caspase-dependent PKR activation and elF2-alpha phosphorylation. In: Journal of Biological Chemistry 276 (45): 41620-41628 (2001). DOI: 10.1074/jbc.M103674200.
41. Sun X. M., Butterworth M., MacFarlane M., Dubiel W., Ciechanover A., Cohen G. M. Caspase activation inhibits proteasome function during apoptosis. In: Molecular cell 14 (1): 81-93 (2004). DOI: 10.1016/s1097-2765(04)00156-x.
42. Kandolf S et al. (2022). Cryo-EM structure of the plant 26S proteasome. In: Plant Commun 3 (3): 100310. DOI: 10.1016/j.xplc.2022.100310.
43. Li Y., Tomko Jr R. J., Hochstrasser M. Proteasomes: Isolation and Activity Assays. In: Current Protocols in Cell Biology 3 (4), e717 (2023). doi.org/10.1002/cpz1.717.
44. Zeenko V. V., Wang C., Majumder M., Komar A. A., Snider M. D., Merrick W. C., Kaufman R. J., Hatzoglou M. An efficient in vitro translation system from mammalian cells lacking the translational inhibition caused by elF2 phosphorylation. RNA. 14(3): 593-602 (2008). doi: 10.1261/rna.825008. Epub 2008 Jan 29. PMID: 18230759; PMCID: PMC2248251.
45. Martinez-Fonts K., Davis C., Tomita T., Elsasser S., Nager A. R., Shi Y, Finley D., Matouschek A. The proteasome 19S cap and its ubiquitin receptors provide a versatile recognition platform for substrates. Nat Commun. 24;11(1): 477 (2020). doi: 10.1038/s41467-019-13906-8. PMID: 31980598; PMCID: PMC6981147.
46. Cabantous S., Terwilliger T. C., Waldo G. S. Protein tagging and detection with engineered self-assembling fragments of green fluorescent protein. Nat Biotechnol. 23(1), 102-107 (2005). doi:10.1038/nbt1044.
47. Manasanch E. E. and Orlowski R. Z. Proteasome Inhibitors in Cancer Therapy. Nat Rev Clin Oncol.; 14(7): 417-433 (2017). doi:10.1038/nrclinonc.2016.206.

## Claims

1. **A method for the preparation of a cell lysate for use in, or as, a cell-free system,** the method comprises the steps of:
**(a)** cultivating and harvesting human cells;
**(b)** optionally, washing said cultured human cells with a washing buffer;
**(c)** admixing the cells with a lysis buffer, wherein the lysis buffer comprises one or more components selected from the group consisting of:
(i) magnesium acetate, preferably in a concentration range between 1 mM and 3 mM;
(ii) an inhibitor of phosphorylation of eIF2α;
(iii) an agonist of dephosphorylation of eIF2α; and
(iv) a caspase inhibitor;
**(d)** lysing the cells by incubating the cells in the admixture of step (c), and preferably applying physical stress to the cells, to obtain a crude cell lysate;
**(e)** purifying the crude cell lysate, such as by removing cellular debris from the crude cell lysate, for example by centrifugation and/or filtration and thereby obtaining an aqueous cell lysate;
**(f)** optionally, cryoprotecting the aqueous cell lysate obtained in (e), for example by admixing the aqueous cell lysate with trehalose, preferably in a concentration range between 1.25 and 5 % (w/v).

2. The method of claim 1, wherein the inhibitor of phosphorylation of eIF2α is a pseudosubstrate for an eIF2α kinase, preferably K3L; optionally wherein the K3L is used in a concentration range between 0.01 mg/mL and 0.03 mg/mL and/or
wherein the agonist of dephosphorylation of eIF2α is growth arrest and DNA damage-inducible protein (GADD34), preferably in a concentration range between 0.01 mg/mL and 0.02 mg/mL.

3. The method of claim 1 or 2, wherein the lysis buffer comprises at least component (iv), preferably (i) and (iv), more preferably (i), (ii) and (iv) and most preferably components (i) to (iv).

4. The method of claim 1, wherein step (d) comprises applying physical force to the cells, preferably wherein the physical force is selected from the group consisting of pressure, freezing and thawing, sonication, impact forces and shearing forces, such as sieving, more preferably, wherein the applying physical force comprises sieving the cells to mesh/sieve and/or passing the cells through a tip of a needle cannula, even more preferably, wherein the mesh/sieve comprises an average pore size of 100 nm to 50 µm, preferably 500 nm to 25 µm, most preferably 1 to 10 µm (such as about 6 µm), even more preferably, wherein the needle cannula has a diameter of less than 2 mm, preferably of less than 1 mm, more preferably of less than 0.5 mm, even more preferably, wherein the method comprises passing the cells through the mesh/sieve and/or needle cannula at least once, preferably at least twice, more preferably for 5 times or more (such as about 7 times through a mesh/sieve and/or about 10 times for a needle cannula).

5. **A method of preparing a cell-free protein synthesis system,** the method comprises a step of providing the aqueous cellular lysate of any one of claims 1 to 4, and adding to the aqueous cellular lysate at least:
**(a)** nucleotide triphosphates (NTPs);
**(b)** an amino acid mixture;
**(c)** one or more of the following components (i) to (v):
(i) magnesium acetate, preferably in a concentration range between 1 mM and 3 mM;
(ii) an inhibitor of phosphorylation of eIF2α;
(iii) an agonist of dephosphorylation of eIF2α;
(iv) a caspase inhibitor
(v) a deubiquitinases (DUB) inhibitor, preferably DUB inhibitor PR619; and/or
**(d)** a nucleic acid construct encoding a protein to be expressed with the cell-free protein synthesis system.

6. The method of claim 5, wherein the nucleic acid construct (d) is added to a final concentration in the cell-free protein synthesis system of 12 nM to 28 nM, preferably 14 nM to 26 nM, more preferably 16 nM to 24 nM, even more preferably 18 nM to 22 nM and/or
wherein the method further comprises a step of adding creatine phosphate, preferably in a concentration range of 15 mM to 45 mM, more preferably 20 mM to 40 mM, even more preferably 25 mM to 35 mM.

7. **A cell-free system obtained by, or obtainable by, performing a method of any one of claims 1 to 6.**

8. The cell-free system of claim 7, wherein the system is a cell-free protein synthesis system and comprises:
**(a)** an aqueous cellular lysate prepared with a method of any one of claims 1 to 4;
**(b)** nucleotide triphosphates (NTPs);
**(c)** an amino acid mixture;
**(d)** an active ubiquitin-proteasome system;
**(e)** one or more of the following components (i) to (v):
(i) magnesium acetate, preferably in a concentration range between 1 mM and 3 mM;
(ii) an inhibitor of phosphorylation of eIF2α;
(iii) an agonist of dephosphorylation of eIF2α;
(iv) a caspase inhibitor;
(v) a deubiquitinases (DUB) inhibitor, preferably DUB inhibitor PR619 and/or
**(f)** a nucleic acid construct encoding a protein to be expressed with the cell-free protein synthesis system.

9. **A method for screening a candidate compound for an effect on a target molecule,** the method comprising:
(a) contacting a candidate compound and a target molecule in a cell-free system of any one of the preceding claims;
(b) determining the amount or level of effected target molecule over time compared to a control or reference,
wherein the effect on the target molecule is selected from the group consisting of a reversible post translational modification, ubiquitin-proteasome dependent protein degradation, lysosome-dependent protein degradation, inhibition of the ubiquitin-proteasome dependent protein degradation, and RNA degradation,
preferably ubiquitin-proteasome dependent protein degradation, wherein an increased or decreased protein stability or protein degradation compared to the control or reference indicates that the candidate compound is a compound having an effect on ubiquitin-proteasome dependent protein degradation of the target molecule.

10. The method of claim 9, wherein the target molecule is contacted with the cell-free system by expression of a target protein by the cell-free system, and wherein the cell-free system is modified by:
**(a)** adding a nucleic acid construct suitable for expressing a target protein to the cell-free protein synthesis system of claim 7 or 8 or optionally adding the target molecule such as a target protein;
**(b)** optionally adding a E3 ubiquitin ligase, a E2 ubiquitin-conjugating enzyme, and/or a E1 ubiquitin-activating enzyme;
**(c)** adding the candidate compound suspected of having an effect on the target molecule,
**(d)** incubating to allow for target protein expression and modification/ degradation,
**(e)** measuring the effect on the target molecule, preferably compared to a control reaction in a cell-free protein synthesis system without a candidate compound.

11. The method of claim 9 or 10, wherein the candidate compound suspected of having an effect on the target molecule comprises PROteolysis TArgeting Chimeras (PROTACs), peptide PROTACs, molecular glues, PhotoPROTAC, Homo-PROTAC, Phosphatase Recruiting Chimera (PhoRC) Phosphorylation Targeting Chimera (PhosTAC), Phosphorylation-Inducing Chimeric Small Molecule (PHIC), Deubiquitinase-Targeting Chimera (DubTAC), Acetylation Tagging System (AceTAG), Lysosome-Targeting Chimera (LYTAC), Ribonuclease Targeting Chimera (RIBOTAC), and inhibitor of the ubiquitin-proteasome dependent protein degradation and/or
wherein the measuring of the effect on the target molecule from step (e) is performed through a method selected from the group consisting of western blot, capillary immunoassay, fluorescence- or luminescence-based reporter assays, mass spectrometry, ALPHA assay, and ELISA assay.

12. **A use of the cell-free protein synthesis system** of claim 7 or 8 for protein expression and/or for investigating ubiquitin-proteasome dependent protein degradation, inhibition of the ubiquitin-proteasome dependent protein degradation, protein stabilization, protein phosphorylation, protein dephosphorylation, protein acetylation, lysosome-dependent protein degradation, or RNA degradation.

13. **A cell-free system kit,** wherein the kit comprises:
**(a)** an aqueous cellular lysate of any one of claims 1 to 4;
**(b)** optionally, additional compounds such as nucleotide triphosphates (NTPs), amino acids, magnesium acetate, GADD34, K3L, caspase inhibitor, DUB inhibitor, creatine phosphate, potassium acetate, DTT, creatine kinase, spermidine, m7GpppG cap analogue, T7 polymerase;
**(c)** and, optionally, instructions directing a user to combine the aqueous cellular lysate and the compounds from the kit with a nucleic acid construct encoding a target protein or a peptide PROTAC.
wherein the aqueous cellular lysate and the compounds are disposed in one or more separate containers.

14. **A cell-free system,** comprising the following components:
(a) magnesium acetate, preferably in a concentration range between 1 mM and 3 mM;
(b) an inhibitor of phosphorylation of eIF2α;
(c) an agonist of dephosphorylation of eIF2α; and/or
(d) a caspase inhibitor;
wherein the cell-free system is prepared from a cell lysate and comprises a functional transcription/translation capability for protein expression and/or a functional ubiquitin-proteasome system for protein degradation.

15. The cell-free system of claim 14, wherein the cell-free system is prepared by a method of any one of claims 1 to 8.
